Europäisches Patentamt

European Patent Office

Office européen des brevets

(19)

(11) EP 0 990 438 A1

(12) EUROPEAN PATENT APPLICATION

(43) Date of publication:
05.04.2000 Bulletin 2000/14

(51) Int Cl.$^7$: A61K 9/08, A61K 33/06,
A61K 47/38, A61P 1/04,
A61P 1/14
// (A61K33/06, 31:426)

(21) Application number: 99307412.9

(22) Date of filing: 20.09.1999

(84) Designated Contracting States:
AT BE CH CY DE DK ES FI FR GB GR IE IT LI LU
MC NL PT SE
Designated Extension States:
AL LT LV MK RO SI

(30) Priority: 21.09.1998 US 157795

(71) Applicant: McNeil-PPC, Inc.
Skillman, NJ 08558-9418 (US)

(72) Inventors:
• Beyerle, Douglas S.
Horsham, PA 19044 (PA)
• Dubek, John J.
Philadelphia, PA 19124 (US)
• McNally, Gerard P.
Strafford, PA 19087 (US)

(74) Representative: Mercer, Christopher Paul et al
Carpmaels & Ransford
43, Bloomsbury Square
London WC1A 2RA (GB)

(54) Heat stable antacid and antigas suspensions

(57) The invention relates to heat stable liquid antacid and/or antigas preparations capable of being pasteurized in the temperature range of 60-100°C comprising one or more acid neutralizing and/or antigas compounds in an aqueous liquid suspension containing hydroxyethylcellulose as suspending agent.

EP 0 990 438 A1

**Description**

BACKGROUND

1. Field of the Invention

The present invention relates to liquid antacid compositions and methods for their preparation. More particularly, the present invention relates to heat stable liquid antacid and antigas preparations containing suspending agents which are capable of being pasteurized without gelling or interacting with the antacid metal salts in the compositions.

2. Description of the Related Art

Gastric antacids are agents that neutralize or remove acid from the gastric contents. Antacids are widely used in the treatment of various gastrointestinal disorders such as peptic ulcers and gastritis. Antacids are also used for the relief of acid indigestion, heartburn, dyspepsia, sour stomach, reflux esophagitis and the like. The clinical use of antacids is based on their ability to neutralize stomach acid and increase the pH of gastric secretions.

Antacids used today are made from a variety of inorganic salts such as calcium carbonate, sodium bicarbonate, magnesium salts and aluminum salts. Magnesium hydroxide and aluminum hydroxide are the most potent magnesium and aluminum salts and are often used in combination. In addition, magnesium oxide, magnesium carbonate, aluminum phosphate, magaldrate, magnesium trisilicate, and aluminum sucrose sulfate (sucralfate) are also employed.

Antigas preparations are those used as adjuncts in the symptomatic treatment of flatulence, functional gastric bloating, and postoperative gas pains. The clinical use of such antigas preparations such as simethicone is based on their antifoam properties. Silicone antifoams spread on the surface of aqueous liquids, forming a film of low surface tension and thus causing the collapse of foam bubbles. This leads to symptomatic relief of pain and distress associated with gas.

Antacids and antigas preparations are available in both liquid suspensions as well as solid dosage forms. In general, liquid suspensions are preferred to tablets or powders since they are more rapidly and effectively solubilized and have a greater ability to react with and neutralize gastric acid or reduce gas. The major benefit of such liquid preparations are their speed of onset. However, a difficulty associated with liquid preparations in general is adequate preservation and terminal sterilization of the finished packaged product. It is key to the preservation of these products that they be packaged into their containers in as microbially clean a state as is possible (i.e. terminally sterilized). This sterilization can be accomplished by either (i) adding chemicals such as sodium hypochlorite or hydrogen peroxide to the products as they are packaged (U.K. Pat. GB 1275885) or (ii) heat treatment (pasteurization) of the liquid antacid.

These sterilization methods suffer from certain disadvantages however. A disadvantage of (i) is that a bad taste is conferred to the product and also it requires that the product be bottled before the chemicals decay which will be a function of how much is initially added. The major concern with (ii) is the effect which heat has on the components of the formulation. One particular problem is the effect of heat on the suspending agents (gums) used to maintain the antacids in suspension. The problem is that the suspending agents tend to undergo thermal gelation at the temperature ranges used in the pasteurization process. The choice of suspending agents available is already limited to those which do not interact with metal ions. Of the suspending agents available, hydroxypropyl methylcellulose (HPMC) is most commonly used and has conventionally been thought to be the best suited because of it's compatibility with antacids (metal ions) and the fact that it could be pasteurized at moderate temperatures 60-70°C.

However, pasteurization in this temperature range is less than ideal. Another problem is that only limited amounts of product can pass through the pasteurizer unit before it becomes blocked with gelled HPMC. Thermal gelation is a known phenomenon associated with HPMC(see for example, A. Haque et al., Thermogelation of Methylcellulose. Part II: effect of hydroxypropyl substituents, *Carbohydrate Polymers* 22 (1993) 175-186). The thermal gelation of the suspending agent in the pasteurization process therefore limits the production capacity and leads to increased costs in manufacturing.

It is therefore the object of this invention to provide a liquid antacid composition which is capable of being pasteurized in the temperature range 60-100°c without gelling or interacting with the divalent and/or trivalent antacid metal salts contained therein.

SUMMARY OF THE INVENTION

The invention relates to heat stable liquid antacid and/or antigas preparations capable of being pasteurized in the temperature range of 60-100°C comprising one or more acid neutralizing and/or antigas compounds in an aqueous liquid suspension containing hydroxyethylcellulose as suspending agent. It has been found that when hydroxyethylcellulose (HEC) is used as a suspending agent for antacid/anti gas suspensions the resulting product has the following properties:

(i) It can be readily sterilized by pasteurization without the concerns of the suspending agent gelling in the pasteurizer. This is not the case with other cellulosic gums such as HPMC,s or HPC.

(ii) In the case of aluminum, calcium or magnesium containing antacid compositions, the HEC does not interact with the trace amounts of free multivalent metal ions (such as $Al^{3+}$, $Ca^{2+}$, or $Mg^{2+}$ cations) in solution as is the case with other suspending agents, e.g. xanthan gum, carrageenan, carbopol, carboxymethylcellulose sodium, alginates, locust bean gum.

(iii) The HEC does not have an inherently high bioburden as do many of the naturally occurring gums such as guar, acacia, tragacanth, locust bean gum etc.

The advantage of using HEC's as suspending agents in antacid and/or antigas suspensions (particularly those containing aluminum, magnesium or calcium containing antacids) allows for easy terminal sterilization of the product by a pasteurization process.

DETAILED DESCRIPTION

The invention relates in particular to liquid antacid and/or antigas preparations comprising an effective amount of one or more acid neutralizing compounds and/or antigas compounds, an hydroxyethylcellulose (HEC) suspending agent, and, optionally, one or more other pharmaceutically acceptable additives. Preferably, the preparation contains 1.0mg - 50mg/5ml HEC suspending agent.

The antacid compounds applicable for use as the active acid neutralizing compound in the present invention are the salts of aluminum, magnesium and calcium conventionally used in liquid antacid compositions. Generally, the composition contains about 200mg-2000mg/5ml acid neutralizing compound. For instance, calcium carbonate in the range of 200 to 2000mg per 5 ml may be employed, as well as magnesium carbonate, magnesium trisilicate, aluminum hydroxide and magnesium hydroxide and mixtures thereof. The amount of antacid in the preparation may conveniently be, for example, in the range of 5% to 35% w/v of the composition. A mixture containing from about 5 to about 15% w/v calcium carbonate and about 2 to about 8% magnesium carbonate or magnesium trisilicate may advantageously be employed. Aluminum hydroxide gel in an amount comprising about 150 to about 800 mg per 5 ml may also be employed. The active acid neutralizing compounds are generally utilized as individual powders, preferably micronized powders.

Alternatively, or in addition to the acid neutralizing compound, the composition may contain an antigas compound such as simethicone as an active ingredient. Such antigas compounds are those used as an adjunct in the symptomatic treatment of flatulence, functional gastric bloating, and postoperative gas pains. For self medication in over-the-counter preparations, antigas compounds such as simethicone are used as an antiflatulent to relieve symptoms commonly referred to as gas, including upper GI bloating, pressure, fullness, or stuffed feeling. Simethicone is often combined with other gastrointestinal medications, such as antacids, antispasmodics or digestive enzymes. The simethicone preferably conforms to the United States Pharmacopoeia (USP XXII) definition, that is, a mixture of fully methylated linear siloxane polymers containing repeating units of the formula $(-(CH_3)_2SiO-)_n$, stabilized with trimethyl siloxy endblocking units of the formula $(-(CH_3)_3-SiO-)$ and silicon dioxide. Other organopolysiloxane antifoam agents are known in the art and may also be used as the active ingredient in this invention. Such organopolylosiloxane antifoam agents are disclosed, for example, in U.S. Patent 5,458,886, and the references discussed therein, hereby incorporated by reference.

The amount of simethicone or other organopolysiloxane antifoam agent contained in the solid oral dosage form should be sufficient to provide a therapeutic dosage to a patient suffering from gas or flatulence and associated symptoms. The preferred dosage range for simethicone is in the range of about 20 mg to about 125 mg per dosage unit, generally not to exceed 500 mg/day. The dosage ranges may vary for age and weight of a patient as well as the severity of symptoms.

The suspending agent is chosen from the hydroxyethylcellulose (HEC) polymers known in the art. HEC is a water soluble polymer derived from cellulose which is nonionic and is therefore unaffected by cations in solution, unlike other ionic cellulose ether polymers.This property makes it ideally suited for use with divalent and/or trivalent cationic antacid salts commonly used. HEC is produced from cellulose by treatment with sodium hydroxide and recation with ethylene oxide to introduce hydroxyethyl groups to yield a hydroxyethyl ether. By performing the reaction under certain conditions, the degree of substitution of hydroxyl groups can be controlled. The solubility in water is achieved as the degree of substitution is increased. HEC is available in various viscosity and solubilty types as NATROSOL® from Aqualon, a division of Hercules Incorporated, Wilmington DE, or as TYLOSE® from Hoechst Celanese Corporation.

In accordance with the invention, HEC is present in the liquid composition as suspending agent in an amount ranging from 1 mg per 5 ml to about 100 mg per 5 ml or about 0.02% w/v to about 2% w/v of the composition.

The liquid antacid suspension preservative component may also advantageously contain a preservative component selected from any pharmaceutically acceptable preservative. The alkyl esters of para-hydroxybenzoic acid (the parabens, e.g. butylparaben, methylparaben and propylparaben) are preferred and may be used alone or in combination. Generally, the parabens are used in a concentration of about 0.02% w/v. Other preservatives include ethylenediamine tetra-acetic acid, propyl-p-hydroxybenzoates, sodium benzoate or sorbic acid.

Advantageously, the liquid antacid preparation may also contain a histamine H2 receptor antagonists or other active agent typically used in gastrointestinal medications. Histamine H2 receptor antagonists are agents which reduce acid secretion and are effective in the treatment of many gastric disorders. Co-administration of histamine H2 receptor antagonists and an antacid is known for example from U. S. Patent No. 5,229,137 and WO 92/00102. Any of the known histamine H2 receptor antagonists may be used such as cimetidine, ranitidine, nizatidine and famotidine. A typical preparation will contain about 100mg to about 400mg of cimetidine, or 50mg to about 150mg of ranitidine or 10mg to 40mg of famotidine per dosage unit (e.g., per 5ml). Typically, the histamine H2 receptor antagonist is employed as the free base or, in the form of the physiologically acceptable salt, such as the hydrochloride salt in the case of ranitidine. Other active agents may be added to the preparation. For instance, analgesics, antidiarrheals, or antispasmodic agents may be added as well as other gastrointestinal agents in dosage amounts conventionally used in the treatment of gastrointestinal dysfunction.

The composition according to the invention, in unit dosage form, may be administered, for example 1 to 4 times per day. The dosage will depend on the active agents that are employed, the condition being treated and the age and weight of the patient. Typical dosages include about 5-30mls of the preparation containing the dose of antacid selected to achieve the desired acid neutralizing effect. A suitable dose range for calcium carbonate is 200mg to 2.0g.

The liquid compositions of the invention are aqueous suspensions containing the active ingredients in admixture with pharmaceutically acceptable excipients typically found in aqueous suspensions for oral administration. Such excipients may be dispersing or wetting agents such as sorbitan esters or lecithin, surface modifiers, aqueous or non-aqueous vehicles such as sorbitol solution, ethyl alcohol or fractionated vegetable oils, or diluents.

The compositions may also contain flavorings, colorants and/or sweeteners as appropriate. Suitable flavorants include fruit flavors, peppermint, licorice or bubble gum flavors. The sweetening agents may be for example bulk sweeteners such as sugars (e.g. sucrose or fructose) or polyols (e.g. maltitol, sorbitol) and/or intense sweeteners such as saccharin, aspartame or acesulfame K.

In addition, the composition may contain buffering agents such as tri or di-ester buffers such as triacetin, or other buffers and buffer salts such as tartaric acid or citric acid.

The liquid antacid compositions of the present invention may be prepared according to conventional techniques well known in the pharmaceutical industry. Thus, for example, the antacid, and the suspending agent may be admixed, if desired, with suitable excipients and dispersed in the aqueous vehicle.

As stated, the use of the HEC suspending agent in the antacid composition of the present invention provides significant advantages in the manufacturing of liquid antacid compositions requiring the use of a suspending agent because terminal sterilization can be accomplished by pasteurization without the concerns of the suspending agent gelling in the pasteurizer. This is not the case with other cellulosic gums such as HPMC,s or HPC. This is demonstrated in the results of the following Example 1 wherein it is shown that the composition of the present invention containing HEC as suspending agent exhibited an increased flow rate in the pasteurization process over the comparative formulation containing HPMC as suspending agent.

In order to further illustrate the present invention and the advantages thereof, the following specific examples are given, it being understood that these examples are intended only to be illustrations without serving as a limitation on the scope of the present invention.

EXAMPLE 1

**Comparing the heat stability of HEC vs. HPMC.**

A recirculation study was conducted on the Plant Scale APV Heat Exchanger. The purpose of this study was to compare the magnitude of fouling (clogging) observed in the heat exchanger with an antacid formula suspended with hydroxypropyl methylcellulose (HPMC) versus an equivalent formula suspended with hydroxyethylcellulose (HEC).

HPMC and HEC are both cellulose based hydrocolloids that enhance the viscosity of a liquid product. Table I highlights the unit formulae used for this study. It should be noted that the RT viscosity (Brookfield spindle #2 speed 12) of these formula are equivalent (225 cps) Two 500 gallon batches were prepared for this study. After processing, both batches were homogenized at 1600psi prior to pasteurization.

Table I

| Raw Material | Formula 1 | Formula 2 |
|---|---|---|
| | mg/5ml | mg/5ml |
| Sorbitol Solution | 953 | 953 |
| Purified Water | 3370.5 | 3381 |
| Hydroxypropyl methylcellulose K4M* | 28.028 | |
| Hydroxyethylcellulose TYLOSE 4000** | | 17.5 |
| Avicel RC 581 | 13 | 10 |
| Simethicone Emulsion (30%) | 73.11 | 73.11 |
| Magnesium Hydroxide Powder | 210.5 | 210.5 |
| Aluminum Hydroxide Gel (13%) | 787.4 | 787.4 |
| Butylparaben | 1 | 1 |
| Propylparaben | 1.5 | 1.5 |
| Creme de Menthe Flavor | 0.233 | 0.233 |
| Lemon Flavor | 18.1 | 18.1 |

\* Dow Chemical

\*\* Hoechst Celanese

Table II illustrates the reduced clogging of the heat exchanger when the present invention using HEC is compared with the equivalent current HPMC formulation. Product was recirculated for 5 minutes then manually drained at ~1.2 gpm for 15 minutes. After draining, the product was recirculated for 5 more minutes and then drained for 15 minutes. This was repeated for 210 minutes. Changes in pump pressure and pump % were monitored throughout the process. An increase in pressure and/or pump % indicates that the heat exchanger is clogging and ultimately would need to be shutdown prematurely.

Table II highlights these results. The results show no significant change in these parameters for the HEC formulation of the present invention, while the HPMC formulation exhibited significant increases in pump pressure and pump %. Overall the results indicate that the HEC formulation shows no evidence of fouling the pasteurizers.

Table II

| Formula 1 (HPMC) | | | |
|---|---|---|---|
| Gallons | Pump Pressure (psi) | Pump % | GPM |
| 0 | 31 | 45 | 1.2 |
| 40 | 54 | 62 | 1.2 |
| 75 | 65 | 70 | 1.2 |
| 100 | 81 | 80 | 1.2 |
| 125 | 94 | 93 | 1.2 |
| Formula 2 (HEC) | | | |
| Gallons | Pump Pressure (psi) | Pump% | GPM |
| 0 | 34 | 62 | 1.2 |
| 40 | 35 | 58 | 1.2 |
| 75 | 35 | 57 | 1.2 |
| 100 | 35 | 59 | 1.2 |

In conclusion, it appears major improvements in product throughput prior to complete fouling of the heat exchanger can be achieved by replacing HPMC in an antacid formula with HEC.

EXAMPLE 2

**Formulation Containing Aluminum Hydroxide Gel.**

|  | Mg/5ml |
|---|---|
| $Al(OH)_3$ Gel (13% $Al_2O_3$) | 1538.5 |
| Sorbitol (70% soln.) | 1000.0 |
| Hydroxyethylcellulose | 15.0 |
| D.I. Water | 3023.5 |
| Flavor | 20.0 |
| Propylparaben NF | 2.0 |
| Butylparaben NF | 2.0 |
|  | 5591.0 |

Place 302.35g. of deionized water and 200g. of 70% sorbitol solution in a 1.5 liter vessel equipped with a IKA mixer. With the agitator set at high speed add 3g. of hydroxyethylcellulose to the water/sorbitol mixture. Continue mixing until all of hydroxyethylcellulose has dissolved. Add 307.7g. of aluminum hydroxide gel to the vessel also under high speed agitation. once the gel has been completely dispersed add the following ingredients in sequence; 0.4g. butylparaben, 0.4g. propylparaben, 4.0g. flavor, finally add 302.35g. of deionized water.
The suspension was then homogenized and pasteurized into plastic Bottles.

EXAMPLE 3

**Formulation Containing $CaCO_3$ Powder and Simethicone.**

|  | mg/5ml |
|---|---|
| $CaCO_3$ powder | 1000.0 |
| Simethicone (30% Emulsion) | 100.0 |
| Sorbitol (70% soln.) | 1000.0 |
| Hydroxyethylcellulose | 15.0 |
| D.I. Water | 3461.0 |
| Flavor | 20.0 |
| Propylparaben NF | 2.0 |
| Butylparaben NF | 2.0 |
|  | 5600.0 |

Place 500.0 g. of deionized water and 200g. of 70% sorbitol solution in a 1.5 liter vessel equipped with a IKA mixer. With the agitator set at high speed add the CaC03 powder. When the $CaCO_3$, has been completely dispersed add 3g. of hydroxyethylcellulose to the mixture. Continue mixing until all of hydroxyethylcellulose has dissolved. Next add the 20g. of simethicone emulsion to the vessel also under high speed agitation. Once the simethicone emulsion has been completely dispersed add the following ingredients in sequence; 0.4g. butylparaben, 0. 4g. propylparaben, 4. Og. flavor, finally add 192. 2g. of deionized water.
The suspension was then homogenized and pasteurized into plastic bottles.

EXAMPLE 4

**Formulation Containing Dihydroxyaluminum Sodium Carbonate (DASC).**

|  | Mg/5ml |
|---|---|
| DASC | 400.0 |
| Sorbitol (70% soln.) | 1000.0 |
| Hydroxyethylcellulose | 25.0 |
| D.I. Water | 3851.5 |
| Flavor | 22.0 |
| Sodium Saccharin | 1.5 |
|  | 5300.0 |

Place 385.15g. of deionized water and 200g. of 70% sorbitol solution in a 1.5 liter vessel equipped with a IKA mixer. With the agitator set at high speed add 5g. of hydroxyethylcellulose to the water/sorbitol mixture. Continue mixing until all of hydroxyethylcellulose has dissolved. Add 80.0g. of DASC to the vessel also under high speed agitation. Once the DASC has been completely dispersed add the following ingredients in sequence; 4.4g. flavor and 0.3g. sodium saccharin, finally add 385.15g. of deionized water.

The suspension was then homogenized and pasteurized into plastic bottles.

EXAMPLE 5

**Formulation Containing Simethicone.**

|  | mg/0.6ml |
|---|---|
| Simethicone (30% Emulsion) | 140.48 |
| Microcrystalline cellulose & Sodium CMC | 0.60 |
| Maltitol | 60.0 |
| Hydroxyethylcellulose | 1.50 |
| D.I. Water | 418.54 |
| Citric Acid | 0.75 |
| Sodium Citrate | 0.30 |
| Sodium Benzoate | 0.60 |
| Flavor | 1.20 |
| Color | 0.03 |
|  | 624.00 |

Place 792.0 Kg. of deionized water to a 2000 liter tank and add 1.136 Kg. of Microcrystalline cellulose. Mix for 5 mins. before adding 2.839 Kg. of HEC, and continue mixing for a further 5 mins. Add the listed ingredients to the batch tank in the following order Citric Acid (1.42Kg.); Sodium Citrate (0.568Kg.); Maltitol (113.6Kg.) and Sodium Benzoate (1.136Kg.). Next add the Simethicone emulsion (266Kg.) to the vessel with good agitation. Once the simethicone emulsion has been completely dispersed add the following separately prepared mixture; 57g. of Colorant and 1.2Kg. flavor dispersed in 10Kg. of deionized water. Once this mixture has been added to the batch tank continue mixing for a further 30 mins.

The suspension was then homogenized and pasteurized into plastic bottles.

**Claims**

1.  A heat stable liquid antacid and/or antigas preparation capable of being pasteurized in the temperature range of 60-100°C comprising one or more acid neutralizing and/or antigas compounds in an aqueous liquid suspension containing hydroxyethylcellulose as suspending agent and, optionally, one or more other pharmaceutically acceptable additives.

2.  The preparation of claim 1, comprising 200 mg to 2000 mg/5ml acid neutralizing compound.

3.  The preparation of claim 1 or claim 2, wherein the acid neutralizing compound is calcium carbonate, dihydroxyaluminium sodium carbonate, magnesium carbonate, magnesium trisilicate, aluminium hydroxide, magnesium hydroxide or a mixture thereof.

4.  The preparation of any one of claims 1 to 3, wherein the acid neutralizing compound is calcium carbonate or aluminium hydroxide gel.

5.  The preparation of any one of claims 1 to 4, further comprising a preservative.

6.  The preparation of any one of claims 1 to 5, containing 1mg to 100 mg/5ml hydroxyethylcellulose.

7.  The preparation of any one of claims 1 to 6, further containing a pharmaceutically effective amount of a histamine H2 receptor antagonist.

8.  The preparation of claim 7, wherein the histamine H2 receptor antagonist is cimetidine, ranitidine, nizatidine or famotidine.

9.  The preparation of claim 8, containing 5 mg to 40 mg of famotidine per 5ml.

10. A method for the terminal sterilization by pasteurization in the temperature range of 60 to 100°C, of liquid antacid and/or antigas preparations containing a suspending agent, which comprises increasing the flow rate by using hydroxyethylcellulose as the suspending agent.

**European Patent Office**

## EUROPEAN SEARCH REPORT

Application Number

EP 99 30 7412

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (Int.Cl.7) |
| P,X | EP 0 872 241 A (MCNEIL PPC INC) 21 October 1998 (1998-10-21) * page 3, line 58 - page 4, line 1; claims 1-10; examples 1-4; tables 1,2 * --- | 1-10 | A61K9/08 A61K33/06 A61K47/38 A61P1/04 A61P1/14 //(A61K33/06, 31:426) |
| X | DATABASE WPI Section Ch, Week 198540 Derwent Publications Ltd., London, GB; Class A96, AN 1985-246145 XP002129414 & JP 60 161915 A (SATO SEIYAKU KK), 23 August 1985 (1985-08-23) * abstract * --- | 1-10 | |
| X | DATABASE WPI Section Ch, Week 198828 Derwent Publications Ltd., London, GB; Class A96, AN 1988-194675 XP002129415 & JP 63 132840 A (FUJI KAGAKU KOGYO KK), 4 June 1988 (1988-06-04) * abstract * ----- | 1-10 | |

TECHNICAL FIELDS SEARCHED (Int.Cl.7)

A61K

The present search report has been drawn up for all claims

| Place of search | Date of completion of the search | Examiner |
|---|---|---|
| MUNICH | 1 February 2000 | Uiber, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 0 990 438 A1**

## ANNEX TO THE EUROPEAN SEARCH REPORT
## ON EUROPEAN PATENT APPLICATION NO.

EP 99 30 7412

01-02-2000

| Patent document cited in search report | | Publication date | Patent family member(s) | | | Publication date |
|---|---|---|---|---|---|---|
| EP 0872241 | A | 21-10-1998 | US | 5914135 | A | 22-06-1999 |
| | | | AU | 6190298 | A | 22-10-1998 |
| | | | CN | 1203079 | A | 30-12-1998 |
| | | | CZ | 9801134 | A | 11-11-1998 |
| | | | HU | 9800876 | A | 28-05-1999 |
| | | | JP | 10316577 | A | 02-12-1998 |
| | | | NZ | 330173 | A | 25-11-1998 |
| | | | PL | 325821 | A | 26-10-1998 |
| JP 60161915 | A | 23-08-1985 | JP | 1496890 | C | 16-05-1989 |
| | | | JP | 63046046 | B | 13-09-1988 |
| JP 63132840 | A | 04-06-1988 | JP | 1958454 | C | 10-08-1995 |
| | | | JP | 6092309 | B | 16-11-1994 |